Europäisches Patentamt

European Patent Office

Office européen des brevets

(19)

(11) Publication number: **0 434 958 A1**

# EUROPEAN PATENT APPLICATION

(21) Application number: **90121814.9**

(22) Date of filing: **14.11.90**

(51) Int. Cl.⁵: **G01N 33/18**

(30) Priority: **28.11.89 US 442014**

(43) Date of publication of application:
**03.07.91 Bulletin 91/27**

(84) Designated Contracting States:
**AT BE CH DE DK ES FR GB GR IT LI LU NL SE**

(71) Applicant: **SPACE BIOSPHERES VENTURE**
**Biosphere 2 Conference Center, Highway 77,**
**96.5 Mile Marker, P. O. Box 689**
**Oracle, Arizona 85623(US)**

(72) Inventor: **Augustine, Margret, Biosphere 2**
**Conference Center**
**Highway 77, 96.5 Mile Marker**
**Arizona 85623(US)**
Inventor: **Leigh, Linda S., Biosphere 2**
**Conference Center**
**Highway 77, 96.5 Mile Marker**
**Arizona 85623(US)**
Inventor: **Hodges, Carl N.**
**4230 East Whittier**
**Tucson, Arizona 85711(US)**

(74) Representative: **Patentanwälte Grünecker,**
**Kinkeldey, Stockmair & Partner**
**Maximilianstrasse 58**
**W-8000 München 22(DE)**

(54) Bio-assay technique for dilute solutions containing toxic substances.

(57) A bioassay technique employing aquatic amphipods sensitive to water borne toxic materials is employed for routine screening of toxicity levels in dilute solutions. For example, a number of Hyalella azteca are counted into a sample of water to be assayed. Similar numbers are counted into volumes of toxicant-free water as a control. Every twenty four hours the number of surviving organisms is counted. The mortality thus determined is compared with the mortality of the same strain of anthropods in solutions with known amounts of toxic materials.

EP 0 434 958 A1

## BIO-ASSAY TECHNIQUE FOR DILUTE SOLUTIONS CONTAINING TOXIC SUBSTANCES

### Field of the Invention

This invention relates to a technique for assaying dilute aqueous solutions for the presence of toxic substances such as residues of agricultural pesticides or the like.

### Background of the Invention

A great concern has developed about release of toxic substances such as pesticides into the environment. Pesticides are of great importance for agriculture, but are hazardous and must often be disposed of in a manner that is environmentally acceptable.

An example is for greenhouses. A variety of plant species are grow in greenhouses where temperature and humidity may be controlled and the plants protected from environmental stresses. A variety of pesticides may be required for maintaining the health and vigor of the plants and preventing their destruction from any of a broad variety of organisms which may be damaging to the species being cultured. It is, therefore, not uncommon to introduce pesticides into a greenhouse by spraying the soil or foliage, or in gaseous form as a fumigant.

Since a greenhouse may remain relatively closed, the pesticides may persist and become a hazard to greenhouse workers. It may, therefore, be desirable to rinse floors, plants and other surfaces in the greenhouse to not only remove pesticides, but also to do the general clean up of leaves, twigs and the like which accumulate in a greenhouse. The water used for rinsing may often contain appreciable amounts of toxic material which should not be discharged into the open environment or ordinary waste disposal systems. Such rinse waters may form dilute solutions of pesticides which must be to disposed of.

To know how to dispose of such solutions, it is important to know the toxicity level. Sophisticated and expensive tests exist for various chemicals that may be in the solutions, but for many purposes, such as day to day preliminary handling and treatment of the solutions, precise knowledge is not needed, and a measure of total toxicity is sufficient without even identifying the specific chemicals present, let alone their concentrations.

It is therefore desirable to provide an inexpensive technique for assaying toxicity of dilute solutions. Such a technique must, however, maintain a level of accuracy and reliability which assures safe handling of such dilute solutions of pesticides.

### Brief Summary of the Invention

There is therefore provided in practice of this invention according to a presently preferred embodiment a technique for assaying water which may contain toxic wastes by immersing a plurality of aquatic amphipods in the water and determining the mortality of the immersed amphipods. This can be compared with the mortality of like amphipods immersed in solutions with known amounts of toxic substances. A preferred amphipod comprising Hyalella azteca.

### Detailed Description

A biological assay, or bioassay, of the toxicity level of aqueous solutions such as greenhouse waste water or the like, uses the mortality of aquatic amphipods such as Hyalella azteca as the measuring instrumentality. The technique is quick, accurate, inexpensive and applicable for a broad variety of toxic materials. what it provides is information on whether the solution being tested is either toxic or non-toxic to sensitive organisms without necessarily identifying which toxic substances may be present. Broadly the technique provides for exposure of standardized bioassay animals to the solution and observing mortality.

It is known that aquatic crustaceans are quite sensitive to the presence of toxic substances in the water in which they live. Aquatic animals within the order amphipoda are sensitive to a range of toxic substances, are small, and reproduce readily. Colonies of the animals may be easily maintained in the laboratory with minimal care or attention. Thus, they provide a suitable animal for bioassay of a broad variety of toxicants. It is particularly preferred to employ fresh water herbivorous amphipods since most requirements for assay are in fresh water, and providing a food source for herbivorous animals is much simpler than trying to maintain a separate population of prey organisms.

A preferred organism for the bioassay is the gammarid amphipod Hyalella azteca. Hyalella azteca is a fresh-water amphipod which is, as are most aquatic crustaceans, highly sensitive to waterborn toxins. They readily reproduce under artificial conditions and require very little attention to maintain a healthy population. They are preferentially herbivorous and are especially fond of the fine root hairs of water hyacinths, Eichornia crassipes.

A Hyalella azteca colony is preferably raised in a plurality of small (e.g. 100 to 125 liter) glass aquaria as a back-up for accidental decimation of a population. Each aquarium is provided with an under gravel filter and aerator. The surface of the water is covered with water hyacinths and about 500 animals may be readily maintained in each such aquarium. Up to 10% of the population may be removed weekly without harm to the population. In the event of over population the food supply may be restricted for suppressing reproduction, or excess animals may be removed and disposed of. Larger aquaria may be used where a large number of assays are being performed and large numbers of animals are needed.

Growth of the water hyacinths is maintained by overhead florescent growth lamps and the aquaria are simply kept at room temperature. In the event growth of water hyacinths in the aquaria is not sufficient for maintaining a desired population of Hyalella azteca, plants may be removed from time to time and replaced with water hyacinths grown in separate tanks.

To perform a bioassay, approximately one liter of water to be assayed is placed in each of a pair of shallow glass dishes. For each test four such dishes are used, two test dishes containing water to be assayed and two control dishes. Each control dish contains approximately one liter of ordinary tap water which has been aged with aeration to avoid thermal shock or chlorine toxicity. Ten adult or sub-adult Hyalella azteca are removed from the aquaria and counted into each dish. A small piece of lettuce (about 25 square centimeters) or similar green plant is placed in each dish for food. It is preferable to cultivate the lettuce specifically for the bioassay laboratory so that it can be certain that it has not been treated at any time before with pesticides. Each dish is equipped with an aerating sparger, covered with a watch glass, and placed on a lighted test rack.

Twenty four hours after stocking the dishes with animals, an accurate count is made of the surviving population of each dish. Each animal is removed from the dish with a small eye dropper or pipette and placed in the watch glass cover. The population number and relative activity level are recorded. The lettuce is removed and replaced with a new piece and surviving animals are returned to the dish after counting.

The counting procedure is repeated for a number of days corresponding to the type of pesticide potentially present in the water. Two days is sufficient for some pesticides such as cypermethrin or permethrin, while four days may be more appropriate for pesticides such as Dichlorvos or Naled.

The approximate toxicity level of the water in the test dishes is calculated by comparison of mortality with mortality observed with predetermined levels of toxicity. The calibration comprises routine determination of the 50% lethal concentration ($LC_{50}$) value for the strain of Hyalella azteca strain being used. Once the $LC_{50}$ value has been determined, the number of observed mortalities in the bioassay procedure is used to back calculate an approximate toxicity level in the water. The $LC_{50}$ level can be determined for a variety of pesticides and should be checked from time to time in the event of changes in the population of Hyalella azteca.

The bioassay derived data for toxicity should also be periodically checked by routine analytical procedures suitable for the pesticides which may be present in the water. Sensitivity in the parts per billion range is required due to the sensitivity of the bioassay animals to very low concentrations of toxic materials. A gas chromatography system with either an electron capture detector or flame photometric detector is the technique of choice since it is relatively simple to operate by a qualified operator and sufficiently sensitive. Such techniques are also consistent with present federally-mandated EPA methods for analysis of many pesticides. Other conventional analytical techniques presently available or developed from time to time may be used for verifying the animal bioassay technique with observed mortality of sensitive aquatic amphipods.

Although described in detail for a specific animal, it will be apparent that the bioassay technique may be employed with other aquatic amphipods sensitive to a variety of toxic materials. It may be desired, for example, to employ parallel tests with different species of amphipods which have differing sensitivities to different toxicants. This may be desirable, for example, where a broad variety of toxic materials may be found in the water to be assayed.

Further, the example employs a fresh water animal, and in the event it is desired to assay sea water, suitable organisms may be substituted. This may be advantageous for example for assaying waters used for mariculture of oysters, abalone, and the like which are also sensitive to presence of toxic substances in the water in which they live. Although specifically mentioned with respect to organic pesticides, it will also be apparent that the technique is applicable for assaying presence of heavy metals or other natural or artificially introduced toxic substances. The technique is suitable for dilute solutions which may contain traces of toxic materials. In the event solutions to be assayed have more than trace amounts of toxicants so that there would be excess mortality of the test amphipods, aliquots may be employed for reducing mortality to meaningful ranges.

Many other modifications and variations will be apparent to those skilled in the art and it will therefore be understood that within the scope of the appended claims the invention may be practiced otherwise than as specifically described.

## Claims

1. A method for assaying water which may contain toxic material comprising the steps of:
   immersing a plurality of aquatic amphipods in water to be assayed;
   determining the mortality of the immersed amphipods; and
   comparing the determined mortality with the mortality of like amphipods immersed in solutions with known amounts of toxic materials.

2. A method as recited in claim 1 comprising the steps of:
   immersing a plurality of the aquatic amphipods in water free of toxicants; and
   determining the mortality of the amphipods in the toxicant-free water as a control comparison.

3. A method as recited in either of claims 1 or 2 wherein the aquatic amphipod comprises Hyalella azteca.

4. A method as recited in any of the preceding claims comprising the steps of:
   counting a plurality of amphipods into water to be assayed and including a food source for the amphipods; and
   counting the surviving amphipods after twenty four hours immersion in the water.

5. A method for assaying water which may contain toxic materials comprising the steps of:
   immersing a known plurality of Hyalella azteca in water to be assayed;
   determining the number of Hyalella azteca surviving after at least one predetermined period; and
   comparing the mortality of the Hyalella azteca with the mortality of the same strain of Hyalella azteca immersed in solutions with known amounts of toxic materials.

6. A method as recited in claim 5 wherein the period is twenty four hours.

7. A method as recited in either of claims 5 or 6 comprising the steps of:
   immersing a plurality of Hyalella azteca in water free of toxicants; and
   determining the mortality of the Hyalella azteca in the toxicant-free water as a control comparison.

European
Patent Office

**EUROPEAN SEARCH
REPORT**

Application Number

**EP 90 12 1814**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | Toxicity Assessment vol. 3, 1988, pages 255 - 269; G.A. Burton et al.: "Evaluation of surrogate tests in toxicant impact assessments" <br> * the whole document * | 1-7 | G 01 N <br> 33/18 |
| X | Environmental Toxicology and Chemistry vol. 5, 1986, pages 933 - 938; A.V. Nebeker et al.: "Survival of Daphnia magna and Hyallela azteca in cadmium-spiked water and sediment" <br> * the whole document * | 1-7 | |
| A | Environmental Research vol. 15, 1978, pages 357 - 367; K.Buehler Fennikoh et al.: "Cadmium toxicity in planctonic organisms of a freshwater food web" <br> * the whole document * | 1-7 | |

| | TECHNICAL FIELDS SEARCHED (Int. Cl.5) |
|---|---|
| | G 01 N |

The present search report has been drawn up for all claims

| Place of search | Date of completion of search | Examiner |
|---|---|---|
| The Hague | 03 April 91 | BOSMA R.A.P. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document